Europäisches Patentamt

**European Patent Office**

Office européen des brevets

(11) Publication number:

**0 255 369**
A2

(12) **EUROPEAN PATENT APPLICATION**

(21) Application number: **87306719.3**

(22) Date of filing: **29.07.87**

(51) Int. Cl.⁴: **A 61 M 25/00**
**A 61 B 6/12**

(30) Priority: **01.08.86 US 891790**

(43) Date of publication of application:
**03.02.88 Bulletin 88/05**

(84) Designated Contracting States:
**AT CH DE FR GB IT LI NL**

(71) Applicant: **ADVANCED CARDIOVASCULAR SYSTEMS, INC,**
**1395 Charleston Road**
**Mountain View, CA 94039-7101 (US)**

(72) Inventor: **Mueller, Richard L., Jr.**
**200 East Dana Street Apartment C62**
**Mountain View California 94041 (US)**

(74) Representative: **Bizley, Richard Edward et al**
**BOULT, WADE & TENNANT 27 Furnival Street**
**London EC4A 1PQ (GB)**

(54) **Catheter tip Marker.**

(57) A catheter tip marker (10) comprises a split sleeve of radiopaque material having an arc length between 180° and 360°. The tip marker (10) is crimped in position about an inner tubular member 16, and an outer tubular member (19) is bonded to the inner tubular member (16), with the tip marker (10) being captured between the two members. The outer tubular member (19) is part of an inflatable balloon (18), and a vent opening (24) extends from the balloon to the distal end of the catheter in alignment with the gap formed between the confronting edges of the marker sleeve.

FIG. 2

EP 0 255 369 A2

**Description**

CATHETER TIP MARKER

This invention pertains to a catheter tip marker, a catheter for use in angioplasty having a fluoroscopically visible tip marker and to a method of manufacturing the same.

Fluoroscopically visible tip markers permit the positions of dilation catheters and the like to be monitored as they are inserted into the vascular system of a patient. Such markers typically consist of one or more bands of radiopaque material mounted on the distal end portion of the catheter. Unfortunately, these bands cannot also be installed without interfering with the other functions of the catheters.

The invention provides a catheter tip marker for identifying the tip of a catheter for use in angioplasty, comprising a split sleeve of radiopaque material having an arc length of more than 180° and less than 360° adapted to be mounted on and crimped about the tip portion of the catheter.

The invention also provides a catheter for use in angioplasty comprising an elongate flexible shaft, an inflatable balloon mounted on the shaft toward the distal end thereof, a vent opening extending from the balloon to the distal end of the shaft, and a marker according to the invention mounted between the balloon and the distal end of the shaft, a longitudinally extending gap between the confronting edges of the marker sleeve being aligned with the vent opening.

The invention further provides a method of manufacturing a dilation catheter having an inner tubular member, an outer tubular member and a marker according to the invention, the method comprising the steps of:
positioning the marker sleeve on the inner member, crimping the sleeve about the inner member to hold it in position, placing the outer member over the inner member and the sleeve, and bonding the outer member and the inner member together, with the sleeve being captured between the two members.

In the accompanying drawings:

Figure 1 is an isometric view of a catheter tip marker;

Figure 2 is a fragmentary isometric view, partly broken away, of a dilatation catheter with a tip marker; and

Figure 3 is a fragmentary centerline sectional view of the catheter shown in Figure 2.

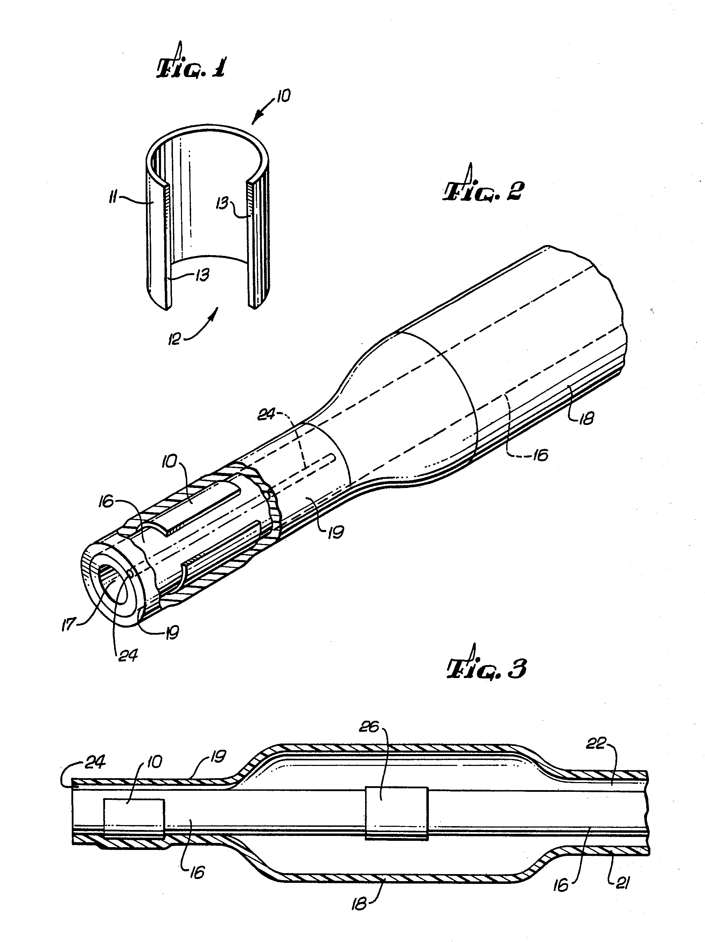

Figure 1 shows a tip marker 10 comprising a sleeve 11 of radiopaque material having an arc length of 270°, with a gap 12 between the confronting longitudinal edges 13 of the sleeve of an arc length of 90°. The sleeve can have any desired arc length between 180° and 360°.

For use in a balloon dilatation catheter, the marker sleeve 11 is fabricated of gold, and it has a length of the order of 0.98 to 1.103mm (0.040 to 0.045 inch), a wall thickness of the order of 0.074mm (0.003 inch) and an internal diameter corresponding to the diamter of the catheter or other device on which the marker is to be mounted. The split sleeve 11 is conveniently fabricated from a cylindrical sleeve by cutting away a portion of the side wall of the sleeve.

The catheter illustrated in Figures 2 to 3 includes an inner tubular member or shaft 16 having an axially extending lumen 17 adapted to receive a guide wire. An inflatable balloon 18 is mounted on the shaft 11 near the distal end of the shaft. The balloon 18 comprises a tubular member which extends the full length of the catheter with a distensible portion. The distal end portion 19 of the outer tubular member of the balloon 18 is bonded to the distal end portion of the inner tubular member 16 to seal the distal end of the balloon chamber. The proximal end portion of the outer tubular member of the balloon 18 is spaced co-axially from the inner tubular member 16 to form an annular passageway 22 for inflating and deflating the balloon.

A small vent opening 24 extends longitudinally between the distal end portion of the balloon 18 and the distal end of the catheter to permit trapped gases to be evacuated from the balloon 18. This opening is of a size large enough to pass gas molecules but small enough to prevent the passage of liquid. This opening typically has a diameter of the order of 0.0147mm (0.0006 inch), and is formed at the junction of the tubular members 16 and 19.

A tip marker 10 of the type illustrated in Figure 1, is provided near the distal end of the catheter. The marker 10 is located between the tubular members 16 and 19, with the vent opening 24 being aligned with the gap between the confronting edges 13 of the marker sleeve 11.

A second marker 26 is mounted on the inner tubular member 16 towards the midpoint of the balloon 18. This marker comprises a band of radiopaque material such as gold.

To manufacture the catheter, the split tip marker sleeve 11 is positioned near the distal end of the inner tubular member 16 and crimped about that member to hold it in position temporarily. The marker 26 is positioned on the inner tubular member 16 and affixed by suitable means such as cement. A longitudinally extending mandrel such as a tungsten wire (not shown) is temporarily affixed to the outer surface of the inner tubular member between the confronting edges 13of the sleeve 11 to form the vent opening 24. The outer tubular member 19 is then positioned over the inner tubular member 16, and the distal end portions of the tubular members 16 and 19 are bonded together by heat sealing to close the distal end of the balloon 18 and to capture the tip marker sleeve 11 between the members. The tungsten wire mandrel is then removed to form the vent opening.

Operation and use of the catheter are as follows. The catheter is positioned in the vascular system of a patient over a guide wire (not shown) which passes through the lumen 17. The position of the tip of the catheter and the balloon 18 can be observed fluoroscopically by meas of the markers 10 and 26. Pressurized fluid is introduced into balloon 18 through the annular passageway 22 to inflate the balloon 18, with any gas trapped in the balloon being expelled through the vent opening 24.

The split marker-sleeve 11 can be used where conventional markers cannot be used. It does not interfere with other functions such as the vent opening for the balloon. Moreover, it can be crimped to hold it in position temporarily while a catheter is being assembled.

## Claims

1. A catheter tip marker (10) for indentifying the tip of a catheter for use in angioplasty, comprising a split sleeve (11) of radiopaque material having an arc length of more than 180° and less than 360° adapted to be mounted on and crimped about the tip portion of the catheter.

2. A marker as claimed in claim 1, wherein the sleeve (11) is of gold.

3. A marker as claimed in claim 1 or 2, wherein the sleeve (11) has an arc length of 270°.

4. A catheter for use in angioplasty comprising an elongate flexible shaft (16), an inflatable balloon (18) mounted on the shaft toward the distal end thereof, a vent opening (24) extending from the balloon (18) to the distal end of the shaft (16), and a marker as claimed in claim 1, 2 or 3, mounted between the balloon (18) and the distal end of the shaft (16), a longitudinally extending gap (12) between the confronting edges (13) of the marker sleeve (11) being aligned with the vent opening (24).

5. A catheter as claimed in claim 4, wherein the distal end portion (19) of the balloon (18) is bonded to the outer surface of the shaft (16), and the marker sleeve (11) is captured in the bond.

6. A method of manufacturing a dilation catheter having an inner tubular member (16), an outer tubular member (19) and a marker as claimed in claim 1, 2 or 3, the method comprising the steps of:
positioning the marker sleeve (11) on the inner member (16), crimping the sleeve (11) about the inner member (16) to hold it in position, placing the outer member (19) over the inner member (16) and the sleeve, and bonding the outer member (19) and the inner member (16) together, with the sleeve being captured between the two members (16 and 19).

7. A method as claimed in claim 6, wherein the two members (16 and 19) are bonded together by heat sealing.

8. A method as claimed in claim 6 or 7 including the steps of positioning a longitudinally extending mandrel on the inner member (16) in the gap (12) between the confronting edges (13) of the sleeve (11), and withdrawing the mandrel after the two members (16 and 19) are bonded together to form a longitudinally extending passageway (24) between the members (16 and 19).

0255369

FIG. 1

FIG. 2

FIG. 3